# EUROPÄISCHE PATENTANMELDUNG

(11) **EP 1 477 809 A1**
(43) Veröffentlichungstag der Anmeldung: **17.11.2004**
(21) Anmeldenummer: 04011393.8
(22) Anmeldetag: 13.05.2004
(51) Int. Cl.: G01N 33/543, B01J 19/00, B01L 3/02, G01N 35/00

(54) **Verfahren zum Drucken von Biomolekülen**

(30) Priorität: 14.05.2003 DE 10321809
(71) Anmelder: Micronas Holding GmbH, 79108 Freiburg (DE)
(72) Erfinder: Klapproth, Holger, 79108 Freiburg (DE)
(74) Vertreter: Stürken, Joachim

(57) **Zusammenfassung**

Die vorliegende Erfindung betrifft allgemein das Gebiet der Biotechnologie. Insbesondere betrifft die Erfindung ein Verfahren zur kontrollierten Aufnahme und Abgabe von Biomolekülen auf feste, halbfeste oder gelartige Oberflächen unter Einwirkung magnetischer Felder.

## Beschreibung

Die vorliegende Erfindung betrifft allgemein das Gebiet der Biotechnologie. Insbesondere betrifft die Erfindung ein Verfahren zur kontrollierten Aufnahme und Abgabe von Biomolekülen auf feste, halbfeste oder gelartige Oberflächen unter Einwirkung magnetischer Felder.

Bereits seit mehreren Jahrzehnten werden Biomoleküle wie insbesondere Nukleinsäuren auf Träger aufgebracht, um sie selbst oder geeignete Bindungspartner untersuchen zu können. Während der Vorgang der kontrolliert dosierten Aufnahme von Biomolekülen aus Vorratsgefäßen lange Zeit mittels Pipette oder vergleichbarer Geräte erfolgte, hat insbesondere die Entwicklung miniaturisierter Systeme und automatisierter Verfahren dazu geführt, dass gerade bei Multiplex-Anwendungen wie der Herstellung von Biomolekülarrays, bei denen Biomoleküle in einer präzisen, rasterartigen Anordnung immobilisiert werden, immer kleinere Mengen einer gewünschten, zumeist wässrigen Probe auf diskrete Positionen geeigneter Oberflächen aufgebracht werden müssen. Die Zuverlässigkeit und Standardisier- sowie Reproduzierbarkeit solcher Systeme lässt jedoch häufig zu wünschen übrig.

Zum einen werden zum Drucken der Biomoleküle Puffersysteme verwendet, die chemisch reaktiv sind und mit den häufig funktionalisierten Oberflächen unerwünschte Reaktionen eingehen. Da zumeist eine kovalente Kopplung von Biomolekülen an Oberflächen angestrebt wird, damit diese auch bei anschließenden Behandlungsschritten an ihren definierten Positionen verbleiben, ist die Auswahl eines geeigneten Druckpuffers von entscheidender Bedeutung für die Bonität des beabsichtigten Untersuchungsergebnisses. Bei sog. Biochips aus Glas werden z.B. Nukleinsäuresonden häufig mittels auf dem Chip befindlicher aminoreaktiver Gruppen in einer nukleophilen Reaktion gebunden. Da die zumeist eingesetzten Puffer ebenfalls nukleophile Eigenschaften aufweisen, kann es zu einer kompetitiven Hemmung der vorgesehenen Kopplungsereignisse kommen, die angesichts der hohen Erwartungen an die Spezifität und Sensitivität derartiger Systeme nicht hingenommen werden kann. Hinzu kommt, dass die Dosiereigenschaften herkömmlicher Zusammensetzungen aufgrund einer zu geringen Viskosität nicht als optimal bezeichnet werden können. Ferner lassen sich bestimmte Biomoleküle (z.B. Insulin) nur schlecht oder gar nicht in den Puffern des Standes der Technik lösen, weshalb eine quantitativ und qualitativ gleichbleibende Druckqualität häufig nicht realisiert werden kann.

Vorrichtungen, die eine hoch parallele Anwendung erlauben, wie DNA-Arrays oder Microarrays mit anderen Biomolekülen werden heute zumeist mit Nadeldruckern oder einer Art Tintenstrahldrucker hergestellt. Dabei spielt das Medium, in dem die Biomoleküle gelöst sind, eine entscheidende Rolle hinsichtlich der Art der Dosierung, der Dosierungsmenge sowie der Form der Tropfen. Sollen nun verschiedene Biomoleküle, für deren Aufnahme kein gemeinsames Lösungsmittel zur Verfügung steht, auf dieselbe Vorrichtung gedruckt werden, steht der Fachmann vor einem Problem, denn die verschiedenen Lösungsmittel weisen auf der zu bedruckenden Oberfläche bezüglich Benetzbarkeit, Kontaktwinkel, Viskosität, Eintrocknungsrate und Oberflächenspannung unterschiedliche Oberflächeneigenschaften auf. Zudem kann es im Falle der Verwendung von Biomolekülen, die in agressiven Lösungsmitteln (z.B. HCl, NaOH) gelöst werden müssen, zur Korrosion der Apparaturen oder zur Beeinträchtigung der Kopplungschemie (z.B. Silane) kommen.

Die Aufgabe der vorliegenden Erfindung liegt daher in der Bereitstellung eines Verfahrens zum Aufbringen bzw. Drucken von Biomolekülen auf eine Oberfläche, mit dem die beschriebenen Nachteile des Standes der Technik überwunden werden.

Die Aufgabe wird erfindungsgemäß durch das Verfahren gemäß Hauptanspruch gelöst. Besondere Ausführungsformen des Verfahrens sind in den Unteransprüchen dargestellt.

Ferner werden erfindungsgemäß ein Verfahren zur Analyse oder Diagnose von Analyten sowie Vorrichtungen zur Durchführung der Verfahren bereitgestellt.

Die vorliegend verwendeten Begriffe "biologische Moleküle" und "Biomoleküle" umfassen jedwede Substanzen und Verbindungen im wesentlichen biologischen Ursprungs, die über Eigenschaften verfügen, welche im Rahmen wissenschaftlicher, diagnostischer und/oder pharmazeutischer Anwendungen relevant sind. Umfasst sind nicht nur native Moleküle, wie sie aus natürlichen Quellen isoliert werden können, sondern auch davon abgeleitete Formen, Fragmente und Derivate, sowie rekombinante Formen und artifizielle Moleküle, sofern sie mindestens eine Eigenschaft der nativen Moleküle aufweisen. Bevorzugte Biomoleküle sind solche, die für analytische, diagnostische und/oder pharmazeutische Zwecke eingesetzt werden können, wie Nukleinsäuren und deren Derivate (DNA, RNA, PNA, LNA, Ribozyme, Oligonukleotide, Plasmide, Chromosomen), Peptide und Proteine (Enzyme, Rezeptorproteine, Proteinkomplexe, Peptidhormone, Antikörper) sowie biologisch aktive Fragmente derselben, Kohlenhydrate und deren Derivate wie insbesondere glykosylierte Proteine und Glykoside, und Fette, Fettsäuren und Lipide.

Für den Fachmann ist klar, dass sich das erfindungsgemäße Verfahren auch auf zelluläre Gewebe und vollständige Zellen sowie Teile derselben (Organellen, Membranen und Membranfragmente etc.) anwenden lässt, sofern sie Träger der obigen Biomoleküle sind. Deshalb sind Gewebe, Zellen und Teile derselben grundsätzlich vom Begriff "Biomoleküle" umfasst.

Das erfindungsgemäße Verfahren zum Drucken einer vorzugsweise vorher festgelegten, definierten Anzahl von Biomolekülen, die über chemisch, enzymatisch und/oder photochemisch spaltbare Crosslinker kovalent an ferromagnetische Partikel gebunden sind, auf eine Oberfläche unter Einwirkung magnetischer Felder, umfasst die folgenden Schritte:
- Magnetfeld-gesteuerte Aufnahme der vorzugsweise definierten Anzahl Partikel-gebundener Biomoleküle mit einer magnetischen oder magnetisierbaren Dosiervorrichtung, und
- Abgabe der aufgenommenen Biomoleküle auf die Oberfläche,
wobei die nach Spaltung der Crosslinker an den Biomolekülen verbleibenden Anteile jeweils mindestens eine funktionelle Gruppe aufweisen, über welche die kovalente Immobilisierung der Biomoleküle auf der Oberfläche erfolgen kann.

Nach einer bevorzugten Ausführungsform erfolgt die Einstellung der Feldstärke des Magnetfeldes, durch welche die Aufnahme der definierten Anzahl von Biomolekülen gesteuert wird, auf der Basis von Normwerten.

Erfindungsgemäß hat sich herausgestellt, dass insbesondere die Nachteile von Systemen, bei denen im wesentlichen flüssige Proben oder Analytenpräparationen gedruckt werden, überwunden werden können, indem die Aufnahme und Abgabe von Biomolekülen, die an ferromagnetische Partikel gebunden sind, mit Hilfe von Magnetfeldern unterstützt wird.

Im Stand der Technik ist die vielfältige Verwendung ferromagnetischer Partikel als Carrier oder Trägersubstanz für Biomoleküle bekannt. Beispielsweise werden von der Fa. Dynal (Norwegen) so genannte "Magnetbeads" in unterschiedlichen Größen und mit verschiedenen Oberflächenmodifikationen angeboten, die je nach Anwendungszweck ausgewählt und erfindungsgemäß eingesetzt werden können (www.dynal.no). Derartige Magnetpartikel weisen z.B. Epoxy-, Amino-, Tosylund/oder Carboxygruppen auf und ermöglichen somit die kovalente Konjugation unterschiedlichster Biomoleküle.

Diese ferromagnetischen Partikel, die selbst nicht permanentmagnetisch sind, werden im Stand der Technik z.B. mittels handelsüblicher Magnete angezogen und können auf diese Weise transportiert oder fixiert werden. Zum Überführen von Proben von einem Gefäß (z.B. Mikrotiterplatte) zu einem anderen werden bereits stabförmige Elektromagnete verwendet. Diese können allerdings nicht im Mikromaßstab eingesetzt werden. Die nachfolgend vorgeschlagenen Dosiervorrichtungen sind demgegenüber jedoch für verschiedene Maßstäbe einsetzbar und erlauben das kontrollierte Drucken von Biomolekülen auf Oberflächen im Kontakt- und Nicht-Kontakt-Verfahren.

Für den Fachmann in Kenntnis der vorliegenden Erfindung ist klar, dass die Verlässlichkeit des erfindungsgemäßen Systems hinsichtlich der exakten Dosierbarkeit entscheidend von Kontrollversuchen bzw. empirischen Daten abhängt, da das Verhältnis zwischen angelegter Feldstärke und Anzahl. (Menge) der an der Dosiervorrichtung gebundenen Partikel zunächst nicht bekannt ist. Weiterhin ist der Belegungsgrad eines gegebenen Partikels mit dem gewünschten Biomolekül zu ermitteln. Kennt man jedoch die Anzahl der Biomoleküle pro Partikel sowie die Beziehung zwischen angelegter Feldstärke und gebundenen Partikeln, kann die Menge der aufgenommenen Biomoleküle verlässlich bestimmt und damit die exakte Dosierung reproduzierbar realisiert werden. Erfindungsgemäß werden diese Parameter und Variablen als Normwerte bezeichnet. Diese Normwerte werden vor der Durchführung des Verfahrens entweder ermittelt oder aus Datenbanken zusammengestellt.

Nach einer bevorzugten Ausführungsform des erfindungsgemäßen Verfahrens wird die Feldstärke des Magnetfeldes der Dosiervorrichtung zum Zeitpunkt der Abgabe der Biomoleküle verringert. Das Magnetfeld kann dabei abgeschaltet oder auf eine so geringe Feldstärke eingestellt werden, dass die beladenen Partikel die Vorrichtung verlassen können.

Nach einer weiteren bevorzugten Ausführungsform sind die Biomoleküle ausgewählt aus der Gruppe bestehend aus Nukleinsäuren und deren Analoga, Peptiden, Proteinen, Multiproteinkomplexen, Sacchariden, Lipiden, und diese einzeln oder in Kombination enthaltenden Verbindungen, Zusammensetzungen und Komplexen.

Die Kopplung der Biomoleküle an die Partikel erfolgt erfindungsgemäß über geeignete Crosslinker, welche chemisch, enzymatisch und/oder photochemisch spaltbar sind. Die Auswahl geeigneter Crosslinker-Moleküle kann der Fachmann angesichts der konkreten Aufgabenstellung leicht vornehmen. Erfindungsgemäß werden Crosslinker eingesetzt, deren nach Abspaltung von den Partikeln an den Biomolekülen verbleibenden Anteile jeweils mindestens eine funktionelle Gruppe aufweisen, über die eine kovalente Immobilisierung der Biomoleküle auf der Oberfläche erfolgen kann. Vorzugsweise wird diese mindestens eine funktionelle Gruppe unter Benzophenon-, Thymidin-, Carbonsäure- und Aminogruppen ausgewählt.

Nach einer weiteren bevorzugten Ausführungsform ist die Oberfläche eine feste, halbfeste oder gelartige Oberfläche. Weiterhin ist die Oberfläche vorzugsweise Teil einer analytischen oder diagnostischen Vorrichtung, wie insbesondere einer Mikrotiterplatte, eines Reaktionsröhrchens oder eines Mikroarrays.

Nach einer alternativen Ausführungsform kann die zu bedruckende Oberfläche zur Sicherung der Abgabe der Partikel-gebundenen Biomoleküle auf vorher festgelegte, definierte Positionen derselben ebenfalls einem Magnetfeld ausgesetzt werden. Dieses Magnetfeld sichert sowohl die Positionierung als auch einen perfekten Druckerfolg, da sämtliche an der Dosiervorrichtung gebundenen Partikel in die gewünschte Position der analytischen Vorrichtung transferiert werden können. Wichtig ist hierbei, dass die Feldstärke des Magnetfeldes der Oberfläche oder die jeweiligen Feldstärken der Magnetfelder unterschiedlicher Oberflächenbereiche zum Zeitpunkt der Abgabe der Biomoleküle höher ist bzw. sind als die Feldstärke des Magnetfeldes der Dosiervorrichtung. Sofern das Magnetfeld der Dosiervorrichtung für die Abgabe abgeschaltet wird, reicht eine vergleichsweise geringe Feldstärke der Oberfläche oder des Oberflächenbereichs zur Realisierung dieses erfindungsgemäßen Vorteils aus. Genaue Angaben zu den benötigten Feldstärken können ermittelt und als Normwerte eingesetzt werden (s.o.).

Das erfindungsgemäße Verfahren zur Analyse oder Diagnose von Analyten basiert auf dem gleichen erfinderischen Prinzip und sieht vor, dass die über chemisch, enzymatisch und/oder photochemisch spaltbare Crosslinker kovalent an ferromagnetische Partikel gebundenen Analyten mittels einer magnetischen oder magnetisierbaren Dosiervorrichtung aufgenommen und auf eine Oberfläche abgegeben werden, wobei die nach Spaltung der Crosslinker an den Analyten verbleibenden Anteile jeweils mindestens eine funktionelle Gruppe aufweisen, über welche die kovalente Immobilisierung der Analyten auf der Oberfläche erfolgen kann.

Nach einer bevorzugten Ausführungsform ist die Oberfläche Teil einer Mikrotiterplatte, eines Reaktionsröhrchens oder eines Mikroarrays. Auch hier ist besonders bevorzugt, dass die Oberfläche zumindest im Bereich der für die Analyse oder Diagnose vorgesehenen Positionen magnetische oder magnetisierbare Eigenschaften aufweist.

Nach einem weiteren Aspekt der vorliegenden Erfindung werden magnetische oder magnetisierbare Dosiervorrichtungen bereitgestellt, mit denen die zuvor beschriebenen Verfahren durchgeführt werden können. Grundsätzlich unterliegt die Auswahl einer erfindungsgemäß geeigneten Dosiervorrichtung keinen Beschränkungen und kann vorzugsweise unter automatisierten Vorrichtungen zum Drucken ausgewählt werden.

Erfindungsgemäß geeignet sind insbesondere elektromagnetische Mikropipetten. Derartige Systeme umfassen mindestens eine ferromagnetische Nadel, welche vorzugsweise Teil eines Elektromagneten ist. Nach einer besonders bevorzugten Ausführungsform ist die mindestens eine Nadel zumindest in dem Bereich, mit welchem die aufzunehmende Probe kontaktiert wird, mit einer inerten Schicht wie z.B. Teflon versehen, um die grundsätzlich gegebene Reaktivität der Nadel zu vermindern. Der Elektromagnet besteht üblicherweise aus einem ferromagnetischen Kern, der von einer stromdurchflossenen Spule magnetisiert wird. Um eine dauerhafte Magnetisierung des ferromagnetischen Kerns zu vermeiden, kann der Elektromagnet nach einer weiteren bevorzugten Ausführungsform ständig oder aber zumindest unmittelbar vor der Abgabe von Partikeln in schneller Folge (z.B. mit 50 Hertz) umgepolt werden.

Ferner werden analytische oder diagnostische Vorrichtungen zur Durchführung der erfindungsgemäßen Verfahren bereitgestellt, deren Oberflächen zumindest im Bereich der für die Analyse oder Diagnose vorgesehenen Positionen magnetische oder magnetisierbare Eigenschaften aufweisen.

Vor dem Hintergrund der vorhergehenden Ausführungen ist klar, dass erfindungsgemäß ferner ein System zum Drucken einer vorher festgelegten, definierten Anzahl von Biomolekülen auf eine Oberfläche unter Einwirkung magnetischer Felder bereitgestellt wird, welches die folgenden Komponenten umfasst:
- Magnetische oder magnetisierbare Dosiervorrichtung zur Aufnahme der definierten Anzahl Partikel-gebundener Biomoleküle,
- Analytische oder diagnostische Vorrichtung, deren Oberfläche zumindest im Bereich der für die Analyse oder Diagnose vorgesehenen Positionen magnetische oder magnetisierbare Eigenschaften aufweist, sowie
- Biomoleküle, die über chemisch, enzymatisch und/oder photochemisch spaltbare Crosslinker kovalent an ferromagnetische Partikel gebunden sind, wobei die nach Spaltung der Crosslinker an den Biomolekülen verbleibenden Anteile jeweils mindestens eine funktionelle Gruppe aufweisen, über welche die kovalente Immobilisierung der Biomoleküle auf der Oberfläche erfolgen kann.

Die Erfindung wird anhand des nachfolgenden Beispiels näher erläutert.

### Beispiel

Streptavidin-beschichtete Magnetbeads von Dynal werden mit einem Oligonukleotid modifiziert, das folgenden Aufbau hat: Biotin, 10 T, EcoRI-Sequenz, 10 T, C6-Aminolinker. Die Menge des eingebauten Oligonukleotids kann durch die Verwendung von 1/100 eines Fluoreszenz-markierten Oligonukleotids ohne die EcoRI-Schnittstelle bestimmt werden. An das Oligonukleotid ist ein weiteres Oligonukleotid hybrisiert, das mit EcoRI-Sequenz und den umliegenden 5 Nukleotiden einen Doppelstrang bildet, welcher mit dem Enzym EcoRI geschnitten werden kann. An diese Aminogruppe des Oligonukleotids wird mittels Sulfo-NHS und EDC (Pierce) der Carboxyterminus eines Antikörpers gebunden. So entstehen Magnetbeads mit gebundenen Antikörpern.

Die Beads werden in einem geeigneten Puffer aufbewahrt. Geeignete Puffer sind z.B. PBS-Puffer, pH8. Bei Verwendung wird die Nadel in das Gefäß mit den Beads eingetaucht, und der Strom der Spule wird eingeschaltet. Die Beads werden von der Nadel angezogen und mit ihrer Hilfe zum Abgabezielort transportiert. Bei hochparallelen Ansätzen bietet sich die Verwendung von geeigneten Pipettierrobotern an. Sobald die Nadel ihre Zielposition erreicht hat, wird die Polarität des Magnetfelds 2 Sekunden lang mit 50 Hertz gewechselt und dann ausgeschaltet. Unter diesen Bedingungen erfolgt das Drucken bzw. die Abgabe sämtlicher Nadel-gebundener Beads. Wahlweise kann das Magnetfeld der Nadel aber auch reduziert werden, so dass nur ein Teil der Beads abgegeben wird. Auf dem Zielort der bedruckten Oberfläche befindet sich ein Puffer, in dem das Enzym EcoRI gelöst ist (geeignete Puffer werden mit den Enzymen geliefert). In diesem Puffer wird die DNA des Crosslinkers gespalten, so dass ein Antikörper mit einem 10 T-Ende freigesetzt wird. Die Magnetbeads können jetzt von der Magnetpipette wieder entfernt werden, sofern dies erwünscht ist. Die Antikörper können jetzt z.B. durch Bindung des 10 T-Endes an ein auf dem Träger fixiertes 10 A Oligonukleotid fixiert werden. Soll die Bindung irreversibel werden, so kann dies durch geeignete Bestrahlung mit UV-Licht erfolgen; bei 260 nm bildet Tymidin Radikale, die an organische Moleküle an der Oberfläche (auch DNA-Moleküle) kovalent binden.

## Patentansprüche

1. Verfahren zum Drucken von Biomolekülen, die über chemisch, enzymatisch und/oder photochemisch spaltbare Crosslinker kovalent an ferromagnetische Partikel gebunden sind, auf eine Oberfläche unter Einwirkung magnetischer Felder, umfassend die folgenden Schritte:
- Magnetfeld-gesteuerte Aufnahme der Partikel-gebundenen Biomoleküle mit einer magnetischen oder magnetisierbaren Dosiervorrichtung, und
- Abgabe der aufgenommenen Biomoleküle auf die Oberfläche,
wobei die nach Spaltung der Crosslinker an den Biomolekülen verbleibenden Anteile jeweils mindestens eine funktionelle Gruppe aufweisen, über welche die kovalente Immobilisierung der Biomoleküle auf der Oberfläche erfolgen kann.

2. Verfahren nach Anspruch 1, bei dem das Drucken eine vorher festgelegte, definierte Anzahl von Biomolekülen betrifft, und bei dem die Einstellung der Feldstärke des Magnetfeldes, durch welche die Aufnahme der definierten Anzahl von Biomolekülen gesteuert wird, auf der Basis von Normwerten erfolgt.

3. Verfahren nach Anspruch 1 oder 2, bei dem die Feldstärke des Magnetfeldes der Dosiervorrichtung zum Zeitpunkt der Abgabe der Biomoleküle verringert wird.

4. Verfahren nach einem der vorhergehenden Ansprüche, wobei die Biomoleküle ausgewählt sind aus der Gruppe bestehend aus Nukleinsäuren und deren Analoga, Peptiden, Proteinen, Multiproteinkomplexen, Sacchariden, Lipiden, und diese einzeln oder in Kombination enthaltenden Verbindungen, Zusammensetzungen und Komplexen.

5. Verfahren nach einem der vorhergehenden Ansprüche, bei dem die mindestens eine funktionelle Gruppe unter Benzophenon-, Thymidin-, Carbonsäure- und Aminogruppen ausgewählt wird.

6. Verfahren nach einem der vorhergehenden Ansprüche, bei dem die Oberfläche eine feste, halbfeste oder gelartige Oberfläche ist.

7. Verfahren nach Anspruch 6, bei dem die Oberfläche Teil einer analytischen oder diagnostischen Vorrichtung, vorzugsweise einer Mikrotiterplatte, eines Reaktionsröhrchens oder eines Mikroarrays ist.

8. Verfahren nach einem der vorhergehenden Ansprüche, bei dem die zu bedruckende Oberfläche zur Sicherung der Abgabe der Partikel-gebundenen Biomoleküle auf vorher festgelegte, definierte Positionen derselben ebenfalls einem Magnetfeld ausgesetzt wird.

9. Verfahren nach Anspruch 8, bei dem die Feldstärke des Magnetfeldes der Oberfläche zum Zeitpunkt der Abgabe der Biomoleküle höher ist als die Feldstärke des Magnetfeldes der Dosiervorrichtung.

10. Verfahren zur Analyse oder Diagnose von Analyten, bei dem die über chemisch, enzymatisch und/oder photochemisch spaltbare Crosslinker kovalent an ferromagnetische Partikel gebundenen Analyten mittels einer magnetischen oder magnetisierbaren Dosiervorrichtung aufgenommen und auf eine Oberfläche abgegeben werden, wobei die nach Spaltung der Crosslinker an den Analyten verbleibenden Anteile jeweils mindestens eine funktionelle Gruppe aufweisen, über welche die kovalente Immobilisierung der Analyten auf der Oberfläche erfolgen kann.

11. Verfahren nach Anspruch 10, bei dem die Oberfläche Teil einer Mikrotiterplatte, eines Reaktionsröhrchens oder eines Mikroarrays ist.

12. Verfahren nach Anspruch 10 oder 11, bei dem die Oberfläche zumindest im Bereich der für die Analyse oder Diagnose vorgesehenen Positionen magnetische oder magnetisierbare Eigenschaften aufweist.

13. Magnetische oder magnetisierbare Dosiervorrichtung zur Durchführung eines Verfahrens nach einem der Ansprüche 1 bis 9 bzw. 10 bis 12.

14. Dosiervorrichtung nach Anspruch 13 zur Durchführung eines Verfahrens nach einem der Ansprüche 10 bis 12, deren Oberfläche zumindest im Bereich der für die Analyse oder Diagnose vorgesehenen Positionen magnetische oder magnetisierbare Eigenschaften aufweist.

15. System zum Drucken einer vorher festgelegten, definierten Anzahl von Biomolekülen auf eine Oberfläche unter Einwirkung magnetischer Felder, umfassend die folgenden Komponenten:
- Magnetische oder magnetisierbare Dosiervorrichtung zur Aufnahme der definierten Anzahl Partikel-gebundener Biomoleküle,
- Analytische oder diagnostische Vorrichtung, deren Oberfläche zumindest im Bereich der für die Analyse oder Diagnose vorgesehenen Positionen magnetische oder magnetisierbare Eigenschaften aufweist, sowie
- Biomoleküle, die über chemisch, enzymatisch und/oder photochemisch spaltbare Crosslinker kovalent an ferromagnetische Partikel gebunden sind, wobei die nach Spaltung der Crosslinker an den Biomolekülen verbleibenden Anteile jeweils mindestens eine funktionelle Gruppe aufweisen, über welche die kovalente Immobilisierung der Biomoleküle auf der Oberfläche erfolgen kann.
